# EUROPEAN PATENT APPLICATION

(11) **EP 4 726 738 A1**
(43) Date of publication of application: **15.04.2026**
(21) Application number: 25208145.0
(22) Date of filing: 10.10.2025
(51) Int. Cl.: G16H 50/50

(54) **IMAGE PROCESSING RELATING TO RETINAL DAMAGE**

(30) Priority: 10.10.2024 GB 202414946
(71) Applicant: Visulise-it Ltd, London EC1V 2NX (GB)
(72) Inventor: ASLAM, Tariq, Wilmslow, Cheshire, SK9 6LZ (GB)
(74) Representative: HGF

(57) **Abstract**

A computer-implemented method and an apparatus for generating a predicted view for a patient is provided. The computer-implemented method comprises receiving a first image indicative of a first view, receiving a retinal image for the patient, the retinal image comprising an image of a scan of the patient's retina including a damaged area, and generating a second image indicative of a predicted view for the patient in dependence on the first image and the retinal image.

## Description

### Technical Field

The present disclosure relates to image processing methods related to retinal damage. Aspects of the invention relate to a computer-implemented method for generating a predicted view for a patient, and to an apparatus to implement the computer-implemented method.

### Background

It is known that patients with conditions or diseases that affect the patient's retina can experience retinal cell disruption, degradation and death. Retinal cell degradation and death may be referred to as retinal atrophy (RA). There are several conditions and diseases that may result in retinal atrophy. One such condition is age-related macular degeneration (AMD), which affects a patient's central vision. Geographic atrophy (GA) is an advanced stage of AMD where the patient experiences damage to the retina in the form of atrophic lesions, which affect the macula, including the fovea. This cellular level damage impacts how the brain processes visual input. Many other diseases can also affect the retina tissues in this way or in different ways.

Image processing can be used to portray an estimated effect of retinal disease and/or a prognosis on a patient's vision by adjusting photographs of common visual scenes. However, it can be challenging to portray the visual impact that retinal cell degradation and death has on a person's sight.

It is an aim of the present invention to address one or more of the disadvantages associated with the prior art.

### Summary of the invention

Aspects and embodiments of the invention provide a computer-implemented method and an apparatus to implement this computer-implemented method, as claimed in the appended claims.

According to an aspect of the disclosure, there is provided a computer-implemented method for generating a predicted view for a patient, the method comprising generating an image indicative of a predicted view for the patient in dependence on a first image, indicative of a first view, and a retinal image comprising a representation of the patient's retina including a damaged area.

In this way, the method of the provides an indication of how damage to a patient's retina may affect their vision now and/or in the future, wherein the indication is specific to the patient.

According to another aspect of the disclosure, there is provided a computer-implemented method for generating a predicted view for a patient, the method comprising: receiving a first image indicative of a first view; receiving a retinal image for the patient, the retinal image comprising a representation of the patient's retina including a damaged area; and generating a second image indicative of a predicted view for the patient in dependence on the first image and the retinal image. Generating the second image comprises: identifying the damaged area in the retinal image; identifying a first area of the first image to be distorted in dependence on the identified damaged area; and distorting the first area of the first image by infilling the first area using pixels from a second area different from the first area.

In this way, the method provides an image modified to indicate the how a patient's vision may be affected by the damage to their retina. The method may provide the modified image to represent the patient's current vision or how the damage to their retina may affect their vision in the future. The image provided by the method, referred to as the second image, is derived in dependence on the patient's own retinal scan and so is specific to that patient. The second image can be useful to those close to the patient to understand their vision, and can be useful to the patient to understand how their vision may be affected in the future.

The representation of the patient's retina may comprise an image of a scan of the patient's retina.

Infilling the first area may comprise inpainting the first area with pixels from the second area. The method may use structural inpainting techniques, geometric inpainting techniques, textural inpainting techniques, and other suitable inpainting techniques.

Generating the second image may comprise smoothing the distorted first area of the first image by applying a first blurring function to the first area. The first blurring function may cause the first area to be blurred by a first blurring factor. The first blurring factor may be selected in dependence on whether the second image is intended to provide a representation of the patient's current vision or a prediction of how their vision may be in the future.

Identifying the damaged area may comprise generating a mask corresponding to at least one damaged region of the retina in the retinal image. The method may comprise identifying an area of the retinal image corresponding to the at least one damaged region of the retina; and generating the mask corresponding to the identified area. The mask may be a binary mask or the like.

Identifying a first area of the first image may further comprise obtaining a retinal focus point associated with the retinal image; rotating the damaged area the about the retinal focus point; and identifying the first area of the first image in dependence on the rotated damaged area. The rotated damaged area may be used as the first area of the first image. Alternatively, the rotated damaged area may be further processed to identify the first area. For example, the rotated damaged area may be enlarged slightly, with the enlarged area being used as the first area.

Obtaining the retinal focus may comprise identifying the centre of the retina within the retinal image. The retinal focus may be identified in dependence on the location of the macula or fovea of the eye, and in some examples the location of the fovea may be the retinal focus.

Obtaining the retinal focus may comprise obtaining a preferred retinal locus (PRL) of the patient. Where damage to a patient's retina includes damage to the macula or fovea, the patient may develop a preferred retinal locus, i.e. a preferred area of their retina for their centre of vision. As such, considering the patient's PRL when considering their vision provides for improved accuracy of the predicted view.

Generating the second image may further comprise identifying first surrounding zone, wherein the first surrounding zone is determined by proportionally enlarging the first area by a first proportional scaling factor; and applying a second blurring function to the first surrounding zone, wherein the second blurring function causes the first surrounding zone to be blurred by a second blurring factor that is less than the first blurring factor.

The method may comprise determining at least one additional surrounding zone by proportionally enlarging the first area by at least one secondary proportional scaling factor, and applying at least one additional blurring function to the at least one additional surrounding zone so as to blur the at least one additional surrounding zone by an additional blurring factor that is less than the second blurring factor.

The first image may comprise a second centre point and a periphery and generating the second image may further comprise applying a general blurring function to the first image, wherein a general blurring factor is substantially zero at the second centre point of the first image and increases towards the periphery of the first image.

Generating the second image may further comprise obtaining a first scale of the retinal image; and scaling the retinal image based on a first set of predefined dimensions.

Generating the second image may further comprise obtaining a second scale of the first image and an expected viewing distance between the patient and a content of the first image; and scaling the first image based on a second set of predefined dimensions.

The method may comprise a Gaussian filter that is used to determine a blurring factor of any of the blurring function.

The method may further comprise obtaining one or more of a set of blurring factors, a set of distortion factors, and a set of darkening factors; and in dependence on the obtained factors, carrying out one or more of the following actions: setting one or more of the first blurring factor, second blurring factor, and additional blurring factor based on the set of blurring factors; adjusting the distorting of the first area by adjusting the infilling of the first area based on the set of distortion factors; and darkening one or more of the first area, at least one surrounding zone, and the second image based on the set of darkening factors. The set of blurring factors, set of distortion factors, and set of darkening factors may be determined in dependence on one or more of a set of symptom data and a set of visual function data.

The method may comprise outputting the second image for display.

The method may comprise displaying the second image to a user via a headset.

Receiving a first image may comprise receiving a video comprising a plurality of image frames. Furthermore, generating the second image may comprise generating a second image for each image frame of a subset of the plurality of image frames to form an output video.

Receiving a video may comprise receiving a live video feed and generating the second image comprises generating a substantially live output video.

According to a yet further aspect of the disclosure, there is provided an apparatus to implement the computer-implemented method.

According to a still further aspect of the disclosure, there is provided an apparatus for generating a predicted view for a patient, the apparatus being configured to receive a first image indicative of a first view; receive a retinal image for the patient, the retinal image comprising a representation of the patient's retina including a damaged area; and generate a second image indicative of a predicted view for the patient in dependence on the first image and the retinal image. Being configured to generate the second image comprises be configured to: identify the damaged area in the retinal image; identify a first area of the first image to be distorted in dependence on the identified damaged area; and distort the first area of the first image by infilling the first area using pixels from a second area different from the first area.

The apparatus may comprise a camera to capture the first image. The apparatus may comprise a display for displaying the second image.

Within the scope of this application, it is expressly intended that the various aspects, embodiments, examples and alternatives set out in the preceding paragraphs, in the claims and/or in the following description and drawings, and in particular the individual features thereof, may be taken independently or in any combination. That is, all embodiments and/or features of any embodiment can be combined in any way and/or combination, unless such features are incompatible. The applicant reserves the right to change any originally filed claim or file any new claim accordingly, including the right to amend any originally filed claim to depend from and/or incorporate any feature of any other claim although not originally claimed in that manner.

### Brief Description of the Drawings

One or more embodiments of the invention will now be described, by way of example only, with reference to the accompanying drawings, in which:
Fig. 1 shows a flow-chart of a method in accordance with an embodiment of the invention;
Fig. 2 shows a flow chart of a method in accordance with further embodiments of the invention;
Fig. 3 shows a block diagram of an apparatus in accordance with an embodiment of the invention;
Fig. 4 shows a retinal image in accordance with embodiments of the invention;
Fig. 5 illustrates an image based on Fig. 4, in accordance with embodiments of the invention;
Fig. 6 illustrates an image based on Fig. 5 in accordance with embodiments of the invention;
Fig. 7 shows a first image in accordance with embodiments of the invention;
Fig. 8 shows a scaled first image, based on Fig. 7, in accordance with embodiments of the invention;
Fig. 9 illustrates a second image, based on Figs. 4 to 8, in accordance with embodiments of the invention;
Fig. 10 illustrates a second image, based on Fig. 9, in accordance with embodiments of the invention;
Fig. 11 illustrates a second image, based on Fig. 10, in accordance with embodiments of the invention;
Fig. 12 illustrates a second image, based on Fig. 9, in accordance with embodiments of the invention;
Fig. 13 illustrates a second image, based on Fig. 12, in accordance with embodiments of the invention;
Fig. 14 illustrates a blurring function in accordance with embodiments of the invention;
Fig. 15 illustrates a second image, based on Figs. 13 and 14, in accordance with embodiments of the invention;

### Detailed Description

Patients with conditions or diseases that affect the patient's retina, including retinal cell degradation and death, can experience loss of vision in areas that correspond to damaged areas of their retina. Patients with Retinal Atrophy (RA), and specifically with Geographic Atrophy (GA), experience cell degradation and death in areas of their retina. Cell degradation and death impacts how the patient's brain processes visual input and results in the patient having impaired/limited vision in the affected areas of the patient's retina. However, the brain compensates for affected retinal cells by employing its existing knowledge to reconstruct the missing piece of the image.

Those working in the area of RA have worked to create representations of how the condition can affect a patient's vision, for example using image processing. This can be a useful tool for both patients and practitioners when treating patients with impaired vision. However, these existing techniques identify the affected areas of a patient's vision and produce an image lacking the affected area(s). The inventor of the present invention noted that this approach does not take into account the actions of the brain compensating for the damaged cells. Nor does it allow for a personalised representation specific for the retinal damage and/or disease in a particular patient. Therefore, these methods of image processing may not produce the most accurate representation of a patient's visual perspective.

The brain can compensate for the degradation of retinal cells by developing access to new pathways between the retina and the brain. As such, the brain makes use of neuroplasticity to compensate for the affected retinal cells by employing its existing knowledge to reconstruct the missing piece of the image. In other words, the brain "fills in" the damaged visual field by using the unaffected and/or less-affected areas of the retina, and their visual input, to create blurring and/or distortion of the visual field associated with the damaged areas of their retina.

By accurately portraying a patient's visual perspective, mapping damage noted from their own retinal scans, a prediction of the degradation of the patient's vision may be obtained. This in turn can provide a better personalised understanding of what treatment options are suitable and what support may benefit the patient. This is important for patients with vision affected by retinal cell degradation and death, RA, or GA, in supporting them and in their treatment of their medical condition.

The inventors have identified that image processing, to depict the effect of retinal damage on a patient's visual perspective, may be improved by taking account of the brain compensating to produce new pathways.

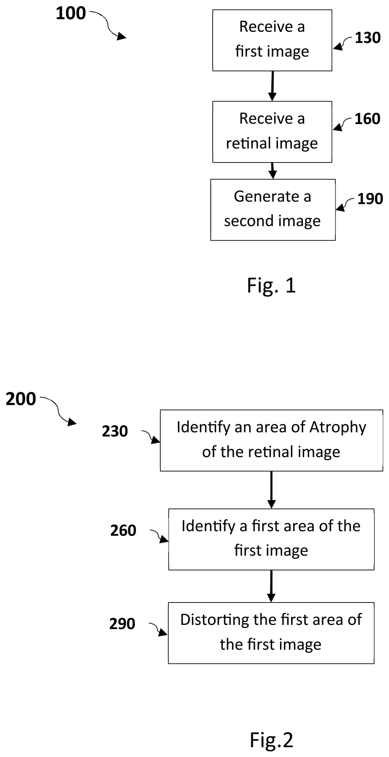

Fig. 1 illustrates a flow diagram of a method, indicated generally by the reference numeral 100, according to an embodiment of the disclosure. The method 100, which may be a computer-implemented method, is for generating a predicted view for a patient with damaged vision. The method 100 comprises generating an image indicative of a predicted view for the patient in dependence on a first image, indicative of a first view, and a retinal image comprising a representation of the patient's retina including a damaged area. In an example, the representation of the patient's retina may comprise an image of a scan of the patient's retina. Fig. 1 shows, in block 130, that the method 100 comprises receiving a first image indicative of a view. In block 160, the method 100 comprises receiving a retinal image for the patient, the retinal image comprising a representation of the patient's retina including a damaged area. In block 190, the method 100 comprises generating a second image indicative of a predicted view for the patient in dependence on the first image and the retinal image. The method 100 may comprise outputting the second image, for example to a display, to a communication module for communication to a user, to a memory for storage, or the like. In this way, the method 100 provides an indication of how a patient's vision may be impacted by the damage to their retina. The 100 method may comprise displaying the second image, for example to the patient and/or the clinician.

Fig. 2 illustrates a flow diagram of a method, indicated generally by the reference numeral 200, according to an embodiment of the disclosure. The method 200, which may be a computer-implemented method, relates to generating a second image indicative of a predicted view for the patient in dependence on the first image and the retinal image. The blocks within the method 200 may be implemented within a corresponding block 190 of the method 100. In block 230, the method 200 comprises identifying the damaged area in the retinal image. In block 260, the method 200 comprises identifying a first area of the first image to be distorted in dependence on the identified damaged area. In block 290, the method 200 comprises distorting the first area of the first image by infilling the first area using pixels from a second area outside the first area. The infilling may comprise inpainting the first area.

The method 100 may comprise a method for generating a predicted view for a patient with retinal atrophy (RA). The method 100 may comprise a method for generating a predicted view for a patient with geographic atrophy (GA). In block 160, the damaged area in the representation of the patient's retina may include an area of atrophy.

The first image, received in block 130, may further comprise data corresponding to one or more of the width of the first image, the distance at which the first image is perceived, and a point of interest. The point of interest may correspond to an object of interest within the first image. The point of interest may comprise at least one of a specific point and a pixel of an object of interest within the first image that the patient would visually focus on, for example a central fixation point. Alternatively, the point of interest may be obtained by automatic selection, such as by image analysis software, or computer implemented measurement and calculation, or by receiving additional data, for example by user selection via a user interface (UI). The first image may be received by retrieving the first image from memory, including local memory. Alternatively, the first image may be received in data received from an external source, such as an external device, a camera, a server, or a database.

The retinal image, received in block 160, may further comprise data corresponding to one or more of a first scale of the retinal image, an outline of the damaged retinal areas within the retinal image, a full segmentation of the damaged retinal areas within the retinal image, a preferred retinal locus point (PRL), and the location of the fovea, within the retinal image. Furthermore, the retinal image may include an image of a scan of the patient's retina where the damaged area includes at least one area of atrophy. The retinal image may be received by retrieving the retinal image from a memory. Alternatively, the retinal image may be received in data received from an external source, such as an external device, a camera, retinal scan equipment, or a database.

In block 230, identifying the damaged area in the retinal image may comprise identifying an area of atrophy therein.

The identified damaged area may include a number of separate areas of the patient's retina, however for convenience, a single area will be referred to herein.

After the damaged area has been identified in block 230, the method 200 may further comprise generating a mask based on the identified damaged area. The generated mask may comprise a region of interest (ROI) corresponding to the damaged area and a background region. The mask may be a binary mask, the ROI corresponding to the area of atrophy having pixel values of "1" and the background region having pixel values of "0", or vice versa.

At block 260, the method 200 may comprise identifying a first area of the first image to be distorted in dependence on the identified damaged area.

At block 260, the method may comprise obtaining a retinal focus point associated with the retinal image, rotating the identified damaged area the about the retinal focus point, and identifying the first area of the first image in dependence on the rotated damaged area. In an example, block 260 of the method 200 may comprise rotating the mask about the retinal focus point, and identifying the first area of the first image in dependence on the rotated mask.

The first area may be identified by mapping or overlaying the identified damaged area onto the first image. The first area may be identified by mapping or overlaying the identified damaged area onto the first image in relation to the retinal focus point and a point of interest, wherein the retinal focus point and the point of interest overlap, are mapped on top of each other, or are made to correspond to the same pixel within the first image.

The retinal focus point may be obtained by identifying a centre of the retina within the retinal image. The retinal focus point may be obtained by identifying a location of the fovea within the retinal image. Alternatively, the retinal focus point may be obtained by obtaining a preferred retinal locus (PRL) of the patient, which may not be limited to the centre of the retina within the retinal image. One or more of a centre of the retina and a PRL may be obtained by automatic selection, e.g., by image analysis software, or computer implemented measurement and calculation. One or more of a centre of the retina and a PRL may be obtained by receiving data corresponding to one or more of a PRL, a centre of the retina, and a location of the fovea, e.g., by user selection via a user interface (UI) or by the retinal image comprising data corresponding to one or more of a PRL, a centre of the retina, and a location of the fovea.

Infilling the first area may comprise inpainting the first area using pixels from a second area outside the first area. The second area may comprise an area of pixels directly adjacent to a periphery of the first area and may not comprise any pixels within the first area. The infilling and/or inpainting may use known techniques as will be apparent to the person skilled in the art.

The method 200 may comprise smoothing the distorted first area of the first image by applying a first blurring function to the first area, wherein the first blurring function causes the first area to be blurred by a first blurring factor.

The method 200 may comprise obtaining a first scale of the retinal image. The method may further comprise scaling the retinal image based on a first set of predefined dimensions. In an example, the retinal image is adjusted to correspond to the first image. This may comprise one or more of scaling, cropping or applying other sizing techniques to the retinal image to fit to the first image. The first scale of the retinal image may be received within a retinal image comprising data corresponding to the first scale of the retinal image. The first scale of the retinal image may be determined automatically, such as by the apparatus performing the method or by an external computing device. The first scale of the retinal image may be obtained by receiving data comprising a first scale of the retinal image, e.g., by user selection via a user interface (UI). The first set of predefined dimensions may be obtained by retrieving the first set of predefined dimensions from a memory. The first set of predefined dimensions may be obtained by receiving data comprising the first set of predefined dimensions, e.g., by user selection via a user interface (UI). The first set of predefined dimensions may be determined based one or more of the first scale of the retinal image, a second scale of the first image, an expected viewing distance between the patient and a content of the first image, a predefined ratio between one or more of the first scale of the retinal image, the second scale of the first image, and the expected viewing distance between the patient and a content of the first image.

The method 200 may comprise obtaining a second scale of the first image. The second scale of the first image may be received within a first image comprising data corresponding to the second scale of the first image. The second scale of the first image may be determined automatically, such as by the apparatus performing the method or by an external computing device. The second scale of the first image may be received in data comprising a second scale of the first image, e.g., by user selection via a user interface (UI). Additionally, the method 200 may further comprise obtaining an expected viewing distance between the patient and a content of the first image. The expected viewing distance may be received within a first image comprising data corresponding to the expected viewing distance. The expected viewing distance of the first image may be determined automatically, such as by the apparatus performing the method or by an external computing device). The expected viewing distance of the first image may be obtained by receiving data comprising the expected viewing distance of the first image, for example by user selection via a user interface (UI).

The methods described herein may comprise changes to the first image beyond those that may be considered to directly correspond to the damaged area identified from the retinal image. A further area of the first image may be altered, wherein the further area may be determined in dependence on the first area. In an example, the further area may be determined in dependence on a determined centre point of the first area, and/or a determined periphery of the first area. The further area may comprise an area of the first image that is concentric with the first area and larger than the first area. The further area may comprise a plurality of surrounding zones. Each surrounding zone may be determined by proportionally enlarging the damaged area by a proportional scaling factor. The method may then comprise applying further blurring functions to the surrounding zones, wherein the blurring function may cause reduced blurring for each successive surrounding zone. In an example, the method may comprise identifying a first surrounding zone and applying a second blurring function to the first one surrounding zone, wherein the second blurring function causes the first surrounding zone to be blurred by a second blurring factor that is less than the first blurring factor. The second blurring function may also be applied to the first area in addition to the first blurring function. In some examples, only certain of the surrounding zones closest to the first area are blurred.

The surroundings zone or zones may be identified in a number of suitable ways, for example, growing outwardly from the periphery of the previous area or zone. For example, the first surrounding zone may grow from the periphery of the first area, while the second surrounding zone may grow from the periphery of the first surrounding zone. The growth may comprise selecting a plurality of pixels surrounding the damaged area such as pixels contiguous to those of the periphery and continuing to include contiguous pixels until the surrounding zone is of a desired size. The desired size may be determined in dependence on a first proportional scaling factor. Determining the surrounding areas in this manner may result in a smoothing effect of irregular edges in the periphery of the first area, which has a more realistic effect on the size and shape of the surrounding zones. The blurring functions may be determined in dependence on a number of considerations, including patient data, such as data derived from the scan and data derived from vision tests. Certain data used may be determined from microperimetry.

The growth may be based on identifying a first centre point of the damaged area, for example a first centre point of an area of atrophy. The first centre point may be a point or a pixel located centrally and within the damaged area. The method 200, may further comprise identifying at least one concentric zone, wherein the at least one concentric zone is determined by proportionally enlarging the damaged area about the first centre point by a first proportional scaling factor.

The surrounding zone may be of substantially the same shape as the area of atrophy and scaled by the first proportional scaling factor, to be proportionally larger than the area of atrophy. The first proportional scaling factor may be obtained by automatic selection or generation. The first proportional scaling factor may be obtained by receiving data comprising one or more scaling factors, e.g., by user selection via a user interface (UI).

A method 100, 200 as described herein may comprise identifying a second centre point and a periphery of the first image. The second centre point may be a point or a pixel located centrally, at an equal distance away from the periphery of the first image, and within the first image. Alternatively, the second centre point may correspond to a point of interest within the first image. The point of interest may correspond to an object of interest within the first image and the specific point that the patient would visually focus on, such as a central fixation point. The periphery of the first image may comprise the outermost pixels of the first image. The method 100, 200 may further comprise applying a general blurring function to the first image, wherein a general blurring factor of the general blurring function is very low, at the second centre point of the first image and increases towards the periphery of the first image. In an example, the blurring functions is zero or close thereto at the second centre point indicative of no or minimal blurring being applied at the second centre point of the first image.

One or more of the first blurring function, second blurring function, and the general blurring function may be implemented by any appropriate function that would smooth the distortion image that they are applied to. In an example, a Gaussian filter may specifically be used to determine a blurring factor of any blurring function.

Additionally, the methods 100, 200, may comprise obtaining one or more of a set of blurring factors, a set of distortion factors, and a set of darkening factors. These factors may be determined by the methods 100, 200, or received from another system etc. The factors may be determined in dependence on a set of symptom data for the patient and/or a set of visual function data for the patient. Symptom data may include data provided by a patient in relation to their experience, and may include information relating to distortion, greying, darkening, and the like of their vision. Visual function data may comprise data related to the visual acuity of a patient and may comprise the results of objective visual measurements from the patient, such as how far down a Snellen chart they can read. The methods 100, 200 may further comprise setting one or more of the first blurring factor, second blurring factor, additional blurring factor, and general blurring factor based on the set of blurring factors. The methods 100, 200 may further comprise adjusting the distorting of the first area by adjusting the infilling of the first area based on the set of distortion factors. The methods 100, 200 may further comprise darkening one or more of the first area, at least one surrounding zone of a further area, and the second image based on the set of darkening factors. Obtaining one or more of a set of blurring factors, a set of distortion factors, and a set of darkening factors may occur prior to block 190 of the method 100 of Fig. 1, and the subsequent actions of setting, adjusting, and darkening may occur within block 190 or within the method 200 of Fig. 2.

Some or all of the set of blurring factors, the set of distortion factors, the set of darkening factors the set of symptom data, and the set of visual function data may be obtained from at least one of a user input, a memory, and a patient record. The user input, memory, and patient record may contain data regarding the health of a patient, which may be specific to the patient's vision comprising data related to the patient's historical retinal scans, the condition of their retina, and a visual perspective of the patient.

The blurring factors of the various blurring functions may be determined using an iterative process and may be determined in dependence on a patient's visual function, including visual acuity.

While the methods 100, 200 described herein are described in relation to a single image, it will be understood that these methods are also applicable to video content comprising a plurality of image frames. In such a case, some or all of the image frames of the video may be treated as the first image of the methods 100, 200, such that the generated second images may form an output video. In an example, the methods 100, 200 may operate so as to appear to a user to provide a real-time output, for example, by outputting the second image on a display, wherein the second image corresponds to a first image being captured approximately at the same time.

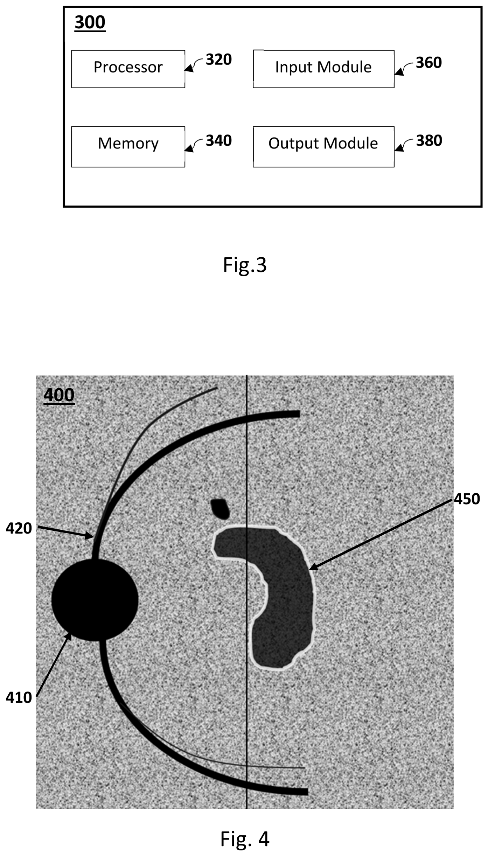

Fig. 3 illustrates an example apparatus 300 in accordance with an embodiment of the present invention. The apparatus 300 is arranged to implement the computer-implemented method(s) discussed herein. The apparatus 300 may comprise one or more processors 320 to execute computer readable instructions to result in the apparatus 300 performing the method(s) discussed herein. The computer readable instructions may be stored on a computer-readable medium, this may be referred to as a non-transitory computer readable medium or a memory means 340. Additionally, the apparatus 300 may comprise one or more input modules 360 for receiving data. The data received by the one or more input modules 360 may comprise at least one of images, user input, patient data, any other form of data comprising information related to at least one of a patient, a retinal scan/image, and an image of an object or scene containing an object. Furthermore, the apparatus 300 may comprise one or more output modules 380 for transmitting data. The data transmitted by the one or more output modules 380 may comprise at least one of images, user input, patient data, any other form of data comprising information related to at least one of a patient, a retinal scan/image, and an image of an object or scene containing an object. The apparatus 300 may be communicatively connected to an external device, such as a server, an external memory, a database, etc. The apparatus 300 may comprise a communication module. The communicative connection may comprise a hardwired or wireless technology, this may refer to USB, Ethernet, Wi-Fi, and telecommunications network, etc.

The apparatus may be configured to receive the first image from a camera in communication with the apparatus 300. The apparatus 300 may comprise a camera configured to capture the first image. The camera may be configured to capture still images and/or video. Such a captured video may comprise a plurality of image frames. The apparatus 300 may comprise a display for displaying a second image generated according to the methods of the disclosure. The display may be configured to display video content.

The apparatus 300 may be communicatively connected to transmit data, for example, the second image, to a display. The apparatus 300 may comprise the display or the display may be external to the apparatus 300.

In an example, the apparatus is in communication with or comprises a wearable device, in particular a device to be worn over a user's eyes. In such an example, the camera may capture video of the view at which the user is looking and the display and provide the corresponding second images to the user overlaying or instead of their view. In this way, such a wearable device may correspond to a virtual reality or augmented reality device, where the display functions as a virtual reality (VR) or augmented reality (AR) display. Such displays may be referred to as a non-immersive display, a semi-immersive display, a fully-immersive display. A fully-immersive display may be referred to as a head mounted display unit, a VR headset, or an AR headset.

Such an apparatus 300 may receive a real time first image, indicative of a real time visual perspective, from a camera associated with a display. The apparatus 300 may function in real time to generate a second image, indicative of a predicted view, based on a real time first image, indicative of a real time visual perspective. That is to say that as the camera changes position and/or orientation the first image is altered, in real time, based on the altered camera position and/or orientation, to result in the second image being altered, in real time, to correspond to the altered camera position and/or orientation. A user wearing such a device would experience vision as predicted based on the retinal image used in generating the second images. Thus, by using their own retinal scan, a patient may be provided an indication of how their vision could be affected by the damage to their retina.

Fig. 4 shows an example received retinal image 400. The retinal image comprises an image of a scan of a patient's retina including a damaged area, which may comprise one or more damaged areas (discussed herein as the damaged area). In the present example, the damaged area is an area of atrophy 450. However, it will be understood that the retinal image 400 of Fig. 4 is just one example, and the teachings of the present disclosure are not limited to the features of the retinal image 400 but are applicable to any retinal image comprising a damaged area such as a diseased area, an area of atrophy, or the like. The retinal image may comprise an optic disc or optic cup 410. The retinal image may comprise one or more of arteries or veins 420. The retinal image may comprise the centre of the retina and a periphery of the retina.

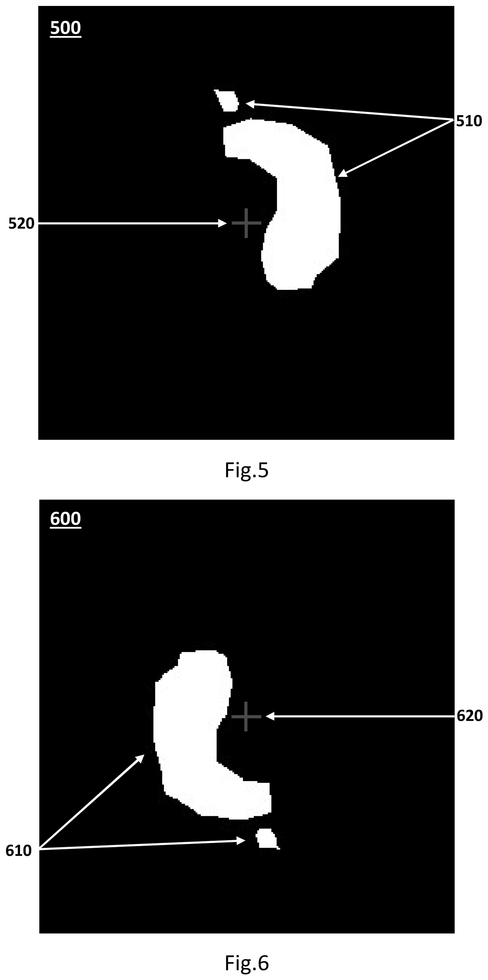

Fig. 5 shows an example identification image 500 based on the retinal image of Fig. 4. The identification image 500 comprises the area of atrophy 510, which correspond to the area of atrophy 450 within the retinal image 400. Fig. 5 shows a retinal focus point 520. The retinal focus point 520 may be obtained and plotted onto the identification image 500 in association with the retinal image 400, and the contents of the retinal image 400. For example, the retinal focus point may be the fovea of the retina. Alternatively, the retinal focus point 520 may be obtained and be associated with the identification image 500 in relation to the area of atrophy 450 of the retinal image 400 or the area of atrophy 510 of the identification image 500. The retinal focus point 520 may not be a visual element of the identification image 500, that is to say that retinal focus point 520 is shown in Fig. 5 only for reference. The identification image 500 may be a mask. The identification image 500 may be a binary mask. If the damaged area overlaps with the fovea, the eye may shift the retinal focus point to a healthier area of the retina. Such a shifted retinal focus point may be referred to as a preferred retinal locus.

Fig. 6 shows an example repositioned identification image 600 based on the retinal image of Fig. 4. The repositioned identification image 600 comprises a repositioned area of atrophy 610, corresponding to the area of atrophy 510 within the identification image 500. The repositioned area of atrophy 610 may correspond to the area of atrophy 510 being rotated 180 degrees about the retinal focus point 520. In this way, the repositioning may be used to compensate for the image inversion that occurs within the eye and brain. The repositioned area of atrophy 610 may correspond to the area of atrophy 510 being inverted in both an X-axis and a Y-axis originating from the retinal focus point 520. The repositioned identification image 600 may comprise a retinal focus point 620. The retinal focus point 620 may be obtained and plotted onto the repositioned identification image 600 in association with one or more of the retinal image 400 and the identification image 500, and the contents of one or more of the retinal image 400 and the identification image 500. Alternatively, the retinal focus point 620 may be obtained and be associated with the repositioned identification image 600 in relation to one or more of the area of atrophy 450 of the retinal image 400, the area of atrophy 510 of the identification image 500, and the repositioned area of atrophy 610 of the repositioned identification image 600. The retinal focus point 620 may not be a visual element of the repositioned identification image 600, that is to say that retinal focus point 620 is shown in Fig. 6 only for reference. The repositioned identification image 600 may be a mask, such as a binary mask.

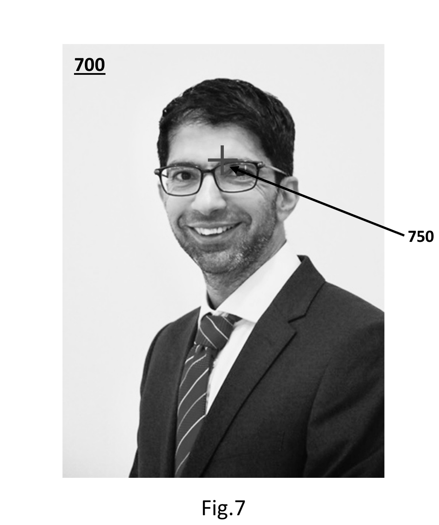

Fig. 7 shows an example received first image 700. The first image 700 comprises a view and may contain an object of interest (not shown). In this example, the first image is a photograph of a man, from mid-torso up. Fig. 7 shows a point of interest 750. The point of interest 750 may correspond to the object of interest, such as a person's face or a specific object, within the first image and the specific visual point or location that the patient would visually focus on, such as a central fixation point. In this example, the point of interest is located at the inner end of one of the man's eyebrows. The point of interest 750 may not be a visual element of the first image 700, that is to say that point of interest 750 is shown in Fig. 7 only for reference. The first image 700 may comprise an image of any shape, size, and content.

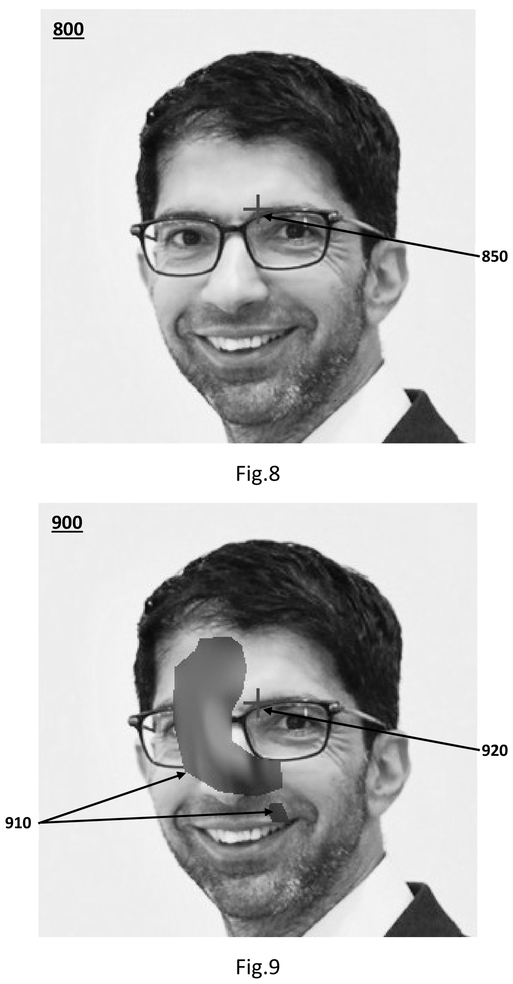

Fig. 8 shows an example of a scaled first image 800. The scaled first image 800 may comprise at least a portion of the first image 700. In the present example, Fig. 8 shows the head of the man shown in Fig. 7. The scaled first image 800 may comprise a portion of the first image that has been scaled based on a first set of predefined dimensions. Fig. 5 shows a point of interest 850 that corresponds to the point of interest 750 shown on the first image 700 in Fig. 7. The point of interest 850 may not be a visual element of the scaled first image 800, that is to say that point of interest 850 is shown in Fig. 8 only for reference).

Fig. 9 shows an example of a second image 900 based on the scaled image 800 and the repositioned identification image 600, prior to distortion. Fig. 9 shows a first area 910 to be distorted, where the first area is overlapped with the second image 900. In the present example, the first area is located such that it overlaps a portion of the man's face, including substantially one eye and most of his nose. Fig. 9 shows a point of interest 950 located on the second image 900. The first area 910 may be identified in dependence on one or more of the area of atrophy 510, the retinal focus point 520, and the point of interest 950. The first area 910 may be identified in dependence on one or more of the repositioned area of atrophy 610, the retinal focus point 620, and the point of interest 950. The first area 910 may be identified by overlaying the identification image 500 or the repositioned identification image 600 onto the scaled first image 800 in dependence on relating the retinal focus point 520 and the point of interest 850. In an example, the first area 910 is identified by aligning the repositioned identification image 600 and the scale first image such that the retinal focus point 620 aligns with the point of interest 850.

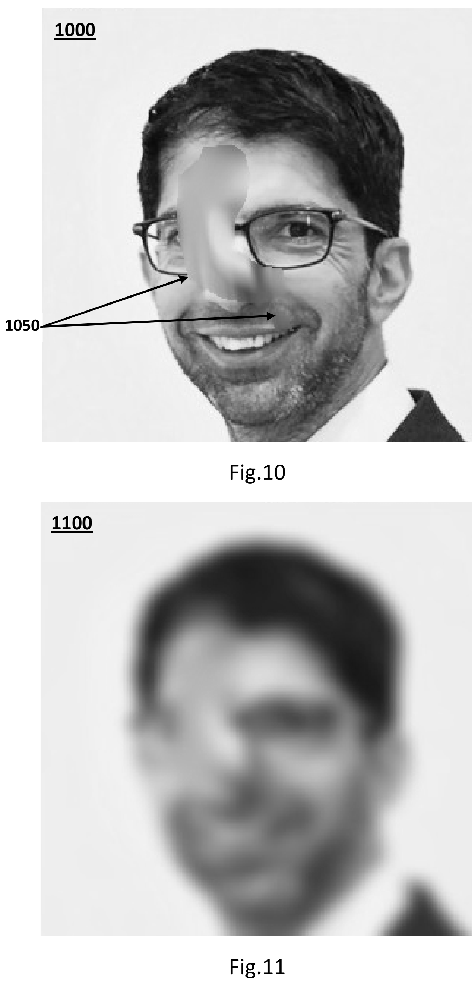

Fig. 10 shows an example of a distorted second image 1000, based on Fig. 9. The distorted second image 1000 may comprise a distorted first area 1050. In the present example, the distorted first area obscures most of one eye and the nose of the man's face. The distorted first area 1050 may be generated by distorting the first area 910 of the first image by infilling the first area 910 using pixels from a second area outside the first area. Infilling the first area 910 may comprise inpainting the first area 910 using pixels from the second area. The second area may comprise an area of pixels directly adjacent to a periphery of the first area and may not comprise any pixels within the first area. The distorted first area 1050 may have a defined periphery, meaning that the distorted first area 1050 may be distinct from and not "smoothed" or "blended" into the remainder of the distorted second image 1000.

Fig. 11 shows an example of the smoothed second image 1100, based on Fig. 10. The smoothed second image 1100 may be generated by smoothing the distorted second image 1000. Fig. 11 shows an example where the whole image has been subject to smoothing. In other examples, only a portion of the distorted second image is subject to smoothing. The smoothing may be performed by applying a first blurring function to the distorted second image 1000, or a portion thereof. The first blurring function causes the distorted second image 1000 to be blurred by a first blurring factor. In an example where only a portion of the distorted second image is smoothed, the portion to be smoothed may correspond to the distorted first area 1050 of the distorted second image 1000. The smoothed second image 1100 may comprise a centre point (not shown) and a periphery.

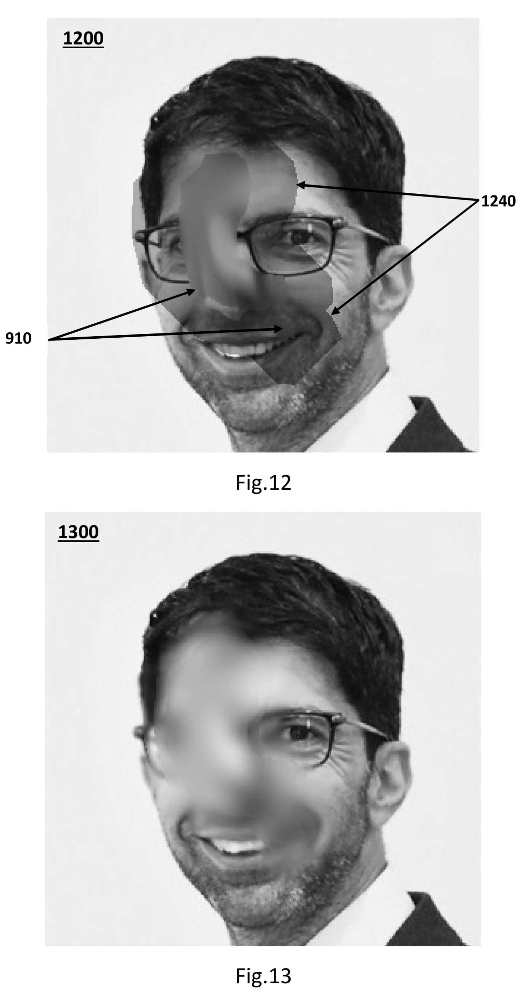

Fig. 12 shows an example of a second image 1200 based on the scaled image 800 and the repositioned identification image 600. The second image 1200 comprises a first area 910 to be distorted, as described herein in relation to Fig. 9 and a further area 1240 to be distorted. The second image 1200 may comprise a point of interest (not shown). The further area 1240 is proportionally larger than the first area and may comprise one or more surrounding zones 1240. In Fig, 12, only one surrounding zone is shown but it will be understood that more than one surrounding zone may be provided. The surrounding zone corresponds in shape to the first area, is proportionally larger than the first area, and is contiguous thereto. Where more than one surrounding zone is included, each is accordingly larger the first area and any surrounding zones between the first area and the surrounding zone in question. The shape of the first surrounding zone is determined in dependence on the shape of the first area, and the shape of any subsequent surrounding zones is determined in dependence on the previous surrounding zone, and thus indirectly in dependence on the first area.

Where a second surrounding zone (not shown) is to be included, it is determined by proportionally enlarging the area of atrophy about the first centre point by at least one secondary proportional scaling factor, larger than the first proportional scaling factor. Any further surrounding zones may be scaled by further proportional scaling factors, each larger than the previous scaling factor.

Where there is a plurality of surrounding zones 1240, they may comprise zones that have areas that overlap each other; may comprise zones that are separate and do not overlap with each other; and may comprise zones that are adjacent to and surrounded by a larger surrounding zone.

Fig. 13 shows an example of a smoothed second image 1300, based on Fig. 12. The smoothed second image 1300 comprises a first area that has been distorted, as described in relation to Fig. 10 and smoothed, as described in relation to Fig. 11.

Additionally, the smoothed second image 1300 comprises a first concentric zone that has been smoothed by applying a second blurring function thereto. The second blurring function may cause blurring by a second blurring factor that is less than the first blurring factor. The second blurring function may cause the at least one concentric zone to be blurred by a second blurring factor that is higher than the first blurring factor. The second blurring function may cause the at least one concentric zone to be blurred by a second blurring factor that is equal to the first blurring factor. The smoothed second image 1300 may comprise at least one concentric zone that has been smoothed by applying a second blurring function to the at least one concentric zone, wherein the second blurring function causes the at least one concentric zone to be blurred by a second blurring factor that is less than the first blurring factor.

Furthermore, the smoothed second image 1300 may comprise at least one concentric zone that has been smoothed, wherein (after the first of the at least one concentric zone 1240) any additional concentric zone (not shown) of the at least one concentric zones 1240 has been smoothed by having an additional blurring function applied to it. The additional blurring function may cause the additional concentric zone to be blurred by an additional blurring factor that is less than the second blurring factor. The additional blurring function may cause the additional concentric zone to be blurred by an additional blurring factor that is higher than the second blurring factor. The additional blurring function may cause the additional concentric zone to be blurred by an additional blurring factor that is equal to the second blurring factor. The smoothed second image 1300 may comprise at least one concentric zone, wherein (after the first of the at least one concentric zone 1240) any additional concentric zone (not shown) of the at least one concentric zones 1240 has an additional blurring function applied to it, which causes the additional concentric zone to be blurred by an additional blurring factor that is less than the second blurring factor.

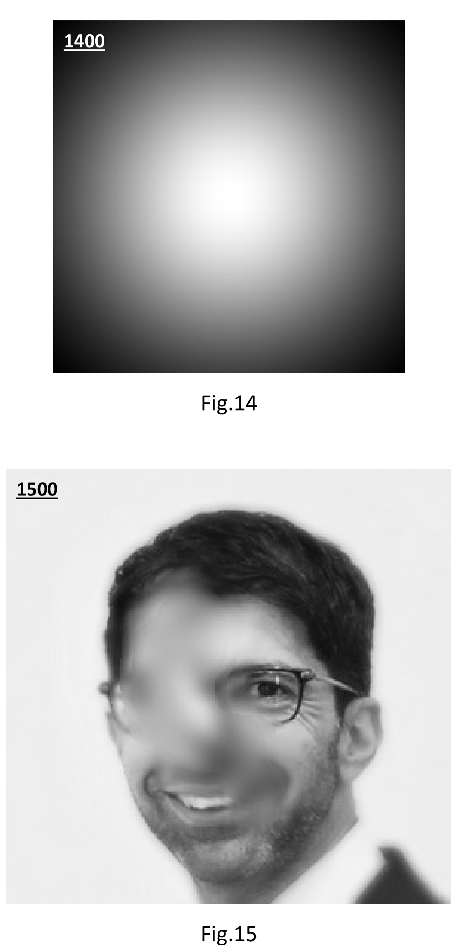

Fig. 14 shows an image of a general blurring function 1400, where the darkness of a pixel indicates the level of blurring applied, where the darker the pixel the higher the blurring factor. The general blurring function 1400 is shown with a central white area transitioning gradually to black the further from centre of the image the pixel is located. The general blurring function 1400 may comprise a low general blurring factor at the centre and an increasing blurring factor towards the periphery of the general blurring function 1400. In the example shown in Fig. 14, the general blurring function 1400 comprises a general blurring factor of zero at the centre, indicative of no blurring being applied at the second centre point of the first image. The general blurring function 1400 be implemented using a Gaussian filter; weighting map; a mask, such as a binary mask, or the like. The general blurring function 1400 may comprise a Gaussian filter to determine the general blurring factor. The general blurring function 1400 may be indicative of the "hill of vision" of a patient, wherein the vision is most accurate at the centre of a visual perspective and reduces in accuracy, or increases in blurring, the further away from the centre of a visual perspective.

Fig. 15 shows a finalized second image 1500, based on Fig. 13. The finalized second image 1500 may comprise the smoothed second image 1300 with a general blurring function 1400 applied to it. The finalized second image 1500 may comprise a centre point (not shown) and a periphery. The finalized second image 1500 is be generated by applying the general blurring function 1400 of Fig. 14 to the smoothed second image 1300, wherein a general blurring factor is zero at the second centre point of the smoothed second image 1300, indicative of no blurring being applied at the second centre point of the first image, and increases towards the periphery of the first image.

Examples disclosed herein aim to recreate the manner in which the brain compensates for damage to the retina and produces new pathways. In this way, the methods and apparatuses described herein provide a more accurate depiction of a how a patient's vision may be affected by their retinal damage. While the example described in relation to Figs. 4 to 15 relates to a patient having an area of atrophy on their retina, it will be understood that that the present invention is not limited to use with patients having an area of atrophy on their retina but may be used with any patient having a damaged area on their retina.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The reader's attention is directed to all papers and documents which are filed concurrently with or previous to this specification in connection with this application and which are open to public inspection with this specification, and the contents of all such papers and documents are incorporated herein by reference.

## Claims

1. A computer-implemented method for generating a predicted view for a patient, the method comprising:
receiving a first image indicative of a first view;
receiving a retinal image for the patient, the retinal image comprising a representation of the patient's retina including a damaged area; and
generating a second image indicative of a predicted view for the patient in dependence on the first image and the retinal image, wherein generating the second image comprises:
identifying the damaged area in the retinal image;
identifying a first area of the first image to be distorted in dependence on the identified damaged area; and
distorting the first area of the first image by infilling the first area using pixels from a second area different from the first area.

2. The computer-implemented method of claim 1, wherein infilling the first area comprises inpainting the first area with pixels from the second area.

3. The computer-implemented method of claim 1 or 2, wherein generating the second image further comprises:
smoothing the distorted first area of the first image by applying a first blurring function to the first area, wherein the first blurring function causes the first area to be blurred by a first blurring factor.

4. The computer-implemented method of any preceding claim, wherein identifying the damaged area further comprises:
identifying an area of the retinal image corresponding to at least one damaged region of the retina; and
generating a mask corresponding to the identified area.

5. The computer-implemented method of any preceding claim, wherein identifying the first area of the first image further comprises:
obtaining a retinal focus point associated with the retinal image;
rotating the damaged area the about the retinal focus point; and
identifying the first area of the first image in dependence on the rotated damaged area.

6. The computer-implemented method of claim 5, wherein obtaining the retinal focus comprises obtaining a preferred retinal locus, PRL of the patient.

7. The computer-implemented method of any preceding claim, wherein generating the second image further comprises:
identifying first surrounding zone, wherein the first surrounding zone is determined by proportionally enlarging the first area by a first proportional scaling factor; and
applying a second blurring function to the at first zone, wherein the second blurring function causes the first surrounding zone to be blurred by a second blurring factor that is less than the first blurring factor.

8. The computer-implemented method of claim 8, comprising determining at least one additional surrounding zone by proportionally enlarging the first area by at least one secondary proportional scaling factor, and applying an additional blurring function to the at least one additional surrounding zone so as to blur the additional surrounding zone by an additional blurring factor that is less than the second blurring factor.

9. The computer-implemented method of any preceding claim, wherein:
the first image comprises a second centre point and a periphery; and
wherein generating the second image further comprises applying a general blurring function to the first image, wherein a general blurring factor is substantially zero at the second centre point of the first image and increases towards the periphery of the first image.

10. The computer-implemented method of any preceding claim, wherein generating the second image comprises:
obtaining a first scale of the retinal image; and
scaling the retinal image based on a first set of predefined dimensions.

11. The computer-implemented method of any preceding claim, wherein generating the second image further comprises:
obtaining a second scale of the first image and an expected viewing distance between the patient and a content of the first image; and
scaling the first image based on a second set of predefined dimensions.

12. The computer-implemented method of any preceding claim, wherein the method further comprises:
obtaining one or more of a set of blurring factors, a set of distortion factors, and a set of darkening factors; and in dependence on the obtained factors, carrying out one or more of the following actions:
setting one or more of the first blurring factor, second blurring factor, and additional blurring factor based on the set of blurring factors;
adjusting the distorting of the first area by adjusting the infilling of the first area based on the set of distortion factors; and
darkening one or more of the first area, at least one surrounding zone, and the second image based on the set of darkening factors.

13. The computer-implemented method of any preceding claim, wherein the method further comprises outputting the second image for display.

14. An apparatus to implement the computer-implemented method of any of the preceding claims

15. An apparatus as claimed in claim 14, comprising a camera to capture the first image and a display for displaying the second image.
